# EUROPEAN PATENT SPECIFICATION

(11) **EP 1 336 088 B1**
(45) Date of publication and mention of the grant of the patent: **10.08.2005**
(21) Application number: 01997697.6
(22) Date of filing: 21.11.2001
(51) Int. Cl.: G01N 15/04, C12Q 1/68, C12N 5/06

(54) **STEP-WISE PROCESS TO RECOVER OR ELIMINATE BIOLOGICAL SUBSTANCES BY FLOTATION FROM UNDERLAYERED COLLOIDAL MEDIUM**
SCHRITTWEISER PROZESS ZUR WIEDERHERSTELLUNG ODER BESEITIGUNG BIOLOGISCHER SUBSTANZEN DURCH FLOTATION AUS EINEM ZUGRUNDELIEGENDEN KOLLOIDALEN MEDIUM
METHODE GRADUELLE DESTINEE A RECUPERER OU ELIMINER DES SUBSTANCES BIOLOGIQUES PAR FLOTTATION A PARTIR D'UN MILIEU COLLOIDAL SOUS-COUCHE

(30) Priority: 22.11.2000 SE 0004271; 22.12.2000 SE 0004777
(43) Date of publication of application: 20.08.2003
(73) Proprietor: Amersham Biosciences AB, 715 84 Uppsala (SE)
(72) Inventor: PERTOFT, Hakan, S-752 40 Uppsala (SE); NORLING, Borje, S-746 93 Balsta (SE)
(74) Representative: Franks, Barry Gerard
(86) International application number: PCT/EP2001/013551
(87) International publication number: WO 2002/042767

(56) References cited:
- WO-A-91/04318
- US-A- 4 927 750
- US-A- 5 437 987
- US-A- 5 962 237

## Description

### Field of the invention

The present invention relates to a method for discontinuous density gradient centrifugation to separate a minimum of two substances in a sample. In the method use is made of colloidal density media and low-density solutions.

### Background of the invention

Separation of cells and cellular components is an important tool within basic research as well as diagnostic and clinical applications. For example, cells and micro-organisms often need to be separated from each other and from different types of samples (e.g. clinical, industrial, food, environmental and forensic samples) in such a way that they can be used for, for example, cultivation, inoculation, cell fusion, clinical therapy and biochemical analysis without interference from inhibiting activities of the sample or inhibiting activities added during the separation process. These inhibiting activities may be, for example, endogenous antibodies, proteases, nucleases, endotoxins, and antibiotics. Micro-organisms, such as bacteria and virus, comprise gene sequences analogous with eukaryotic gene sequences and this may lead to an erroneous result if both of these are present simultaneously in a sample.

To solve the above mentioned separation problems, use has been made of differential centrifugation, filtration, affinity purification, and selective cultivation media. Density media, of both the low molecular weight (Nycodenz etc.) and of the colloidal type (BactXtractor, Percoll, PureSperm) has been used. In the majority of the known processes the separation is based on the density of the inherent components in the used density media after centrifugation in a continuous density gradient. These processes have the drawback that undesired substances are distributed over the whole gradient, and thus also in the desired fraction.

In conventional discontinuous density gradient centrifugation, the sample is loaded on the density media and the desired fraction is recovered from the bottom part of the resulting lower phase. The centrifugation is performed in an angle centrifuge and this means that the bacteria, or other desired cells or fractions, may be harmed when they hit the wall of the centrifuge tube during the sedimentation down to their density level and this may lead to bacterial death and leakage of their DNA/RNA content. When the sample is loaded on the colloidal media there is a risk of contamination of the underlying phases. This contamination is partly due to wall effects, i.e. the sample flows down along the tube wall and may be mixed with underlying phases, and partly due to too rapid, especially initially, loading of the sample leading to admixture with underlying phases. Irrespective of whether the sought fraction after centrifugation is recovered by being aspirated through the upper phase (by a Pasteur pipette or cannula) or if it is recovered after the upper phase has been aspirated away, this procedure leads to a recontamination of the desired fraction. In the first case, a microscopic amount of material is brought from the upper phase to the lower phase by the Pasteur pipette/cannula. In the latter case, contamination occurs because the material in the upper phase cannot be completely removed since it exists in the liquid that is being retained on the inner walls of the tube. This liquid will run downwards when the upper phase is aspirated, and when the upper phase is removed this liquid will contaminate the lower phase. Another drawback with this type of sedimentation separation is that the yield of samples which contain material forming a plug (physical barrier) in the interface between the upper and the lower phase is adversely affected because cells/bacteria are prevented from sedimenting downwards. Another drawback of several density gradient media is their inherent toxicity, which is highly undesirable in many applications.

FR 2 561 256 describes a method of purification of biological particles, such as antigen core particles, using flotation ultracentrifugation on a density gradient. More specifically, the sample is placed on top of a preformed density gradient, which is then centrifuged to allow for collection of the sample in the top layer while contaminants etc have migrated to the lower layers of the gradient. Before this purification process, the sample needs to be concentrated and prepurified. The density gradient is provided by a low molecular compound, namely cesium chloride. The g-force required during the centrifugation in this process is at least 20 000 x g. This high g-force results from the nature of the gradient, where the sedimentation of the gradient solution is balanced by diffusion of solute molecules in the direction opposite that of sedimentation, which is formed by diffusion of salt. Salt gradients have been shown to be less easy to set at exact values than other gradients, such as colloidal silica. Thus, the gradient suggested in FR 2 561 256 will not be stable in time. Furthermore, the osmotic pressure caused by such small molecules as CsCl can be harmful to certain materials, and also interact with biological materials and affect the native characteristics of the sample. Accordingly, this kind of method will not be suitable for separation of more delicate biological structures, such as cells.

**US A 5 437 987** relates to a triple gradient process with antibody panning to recover nucleated fetal cells from maternal blood. This document teaches use of multiple layers of density gradient medium.

**US A 4 927 750** relates to a cell separation process involving centrifugation. This document describes separation of cells from various biological specimen and is not concerned with flotation of one or more desired substances to an uppermost solution of low density.

**US A 5 962 237** relates to a method of enriching rare cells using centrifugation. The centrifugation steps may be repeated for enrichment purposes, ie during the same time and same g-force. This document does not teach flotation of different desired substances.

**WO 91 04318A** relates to separation of different living cells using low molecular solutions which will diffuse into each other and form a continuous or discontinuous gradient.

**US A 4 971 801** relates to a biological response modifier comprising natural membrane vesicles and ribosomes in a suspending buffer. Product isolation is described using gradient centrifugation. The desired fraction is pelleted, ie collected at the bottom of the centrifuge tube.

Finally, also non-ionic gradient media, e.g. sucrose gradients have also been suggested for separation. However, the nature of sugars such as sucrose is similar to the above discussed salts, and accordingly these methods will entail the same sort of drawbacks as mentioned above, i.e. unstable gradients wherein the boundaries become more and more diffuse with time, an osmotic pressure that can harm micro-organisms, cells etc.

### Summary of the invention

One object of the present invention is to avoid one or more of the above drawbacks by providing a new method for discontinuous density gradient centrifugation which relies on a new principle, namely allowing the desired substances to float after one or more centrifugation steps.

A further object of the invention is to provide a method of discontinuous density gradient centrifugation, which includes less risk of contamination of the desired substance(s) than prior art methods. This is achieved by a step-wise method, wherein a lower part of a centrifuged sample is passed over to one or more subsequent steps, repeating the same round of mixing and centrifugation as defined in claim 1 but at different densities and at different g-forces. Since the lower part is further processed, the actual sample requires no pipetting between steps, which eliminates one common way of contamination. In addition, the novel step-wise method provides a superior flexibility as compared to the prior art, since a series of rounds can be designed, each round being performed during conditions specifically separating substances that are known or suspected to be present in the sample.

### Brief description of the drawing

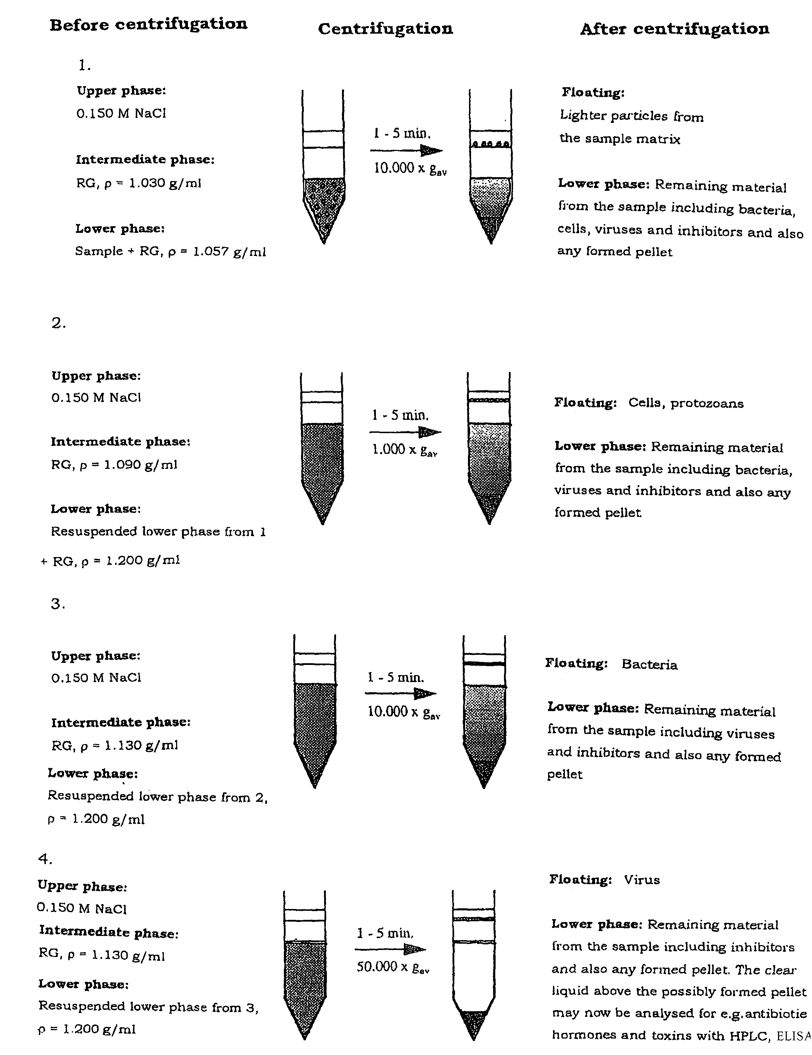

Fig. 1 represents a schematic view of the working principle of the method according to the present invention using four centrifugation steps.

### Detailed description of the invention

Thus, in a first aspect the invention provides a method of discontinuous density gradient centrifugation for separating a minimum of two substances from each other in a sample. The method is characterised by the subsequent steps of
(i) mixing the sample with a high density medium to obtain a specific density of the mixture,
(ii) placing
   a) a first mixture of a high density colloidal centrifugation media and said sample containing said substances at the bottom of a centrifuge tube, in which mixture the density is defined,
   b) a solution of low density on top of the mixture in a)
(iii) centrifuging said tube under a specifically defined g-force so that the desired substance(s) or solute(s) in said sample are allowed to float to the low density solution whereas other substance(s) in said sample will remain in the bottom denser media,
(iv) collecting separately the desired substance(s),
wherein the centrifugation medium is a colloidal density gradient medium and the discontinuous gradients are created by the different layers.

As mentioned above, the above described round of steps (i)-(iv) can be repeated, such as one, two, three or any number of times, including a second, third, fourth etc mixture of a defined density, and centrifuged at a second, third, fourth etc g-force. The skilled person in this field can select the appropriate combinations of g-forces and densities based on the densities of the substance(s) that are to be separated from the sample. Such a systematic variation of g-forces has not been suggested in the context of density gradient centrifugation in the prior art. Also, the advantages of using a colloidal centrifugation medium to this end were quite unexpected, since a method designed according to the present invention can provide purities that are superior to the results of prior art methods applied to the wide variety of samples that the present invention is useful with.

In one embodiment, a colloidal density gradient centrifugation medium of a lower density than the mixture in a) is placed between the mixture in a) and the low-density solution in b). This gives an extra barrier between the mixed sample and the low-density solution and improves and facilitates the recovery following separation. In the present context, the term "discontinuous" refers to a successive layering of solutions of different density e.g. in a centrifuge tube.

The colloidal nature of the centrifugation medium is an essential feature of the invention and will provide advantages such as a much improved stability of the density gradient. The centrifugation medium used according to the invention should be a heavy medium, such as a colloidal silica-based material. The medium should be inert, autoclavable in the presence of salt, have a low or no endotoxin level, an as low osmotic pressure as possible, preferably below 20 mOsm/kg, a low viscosity in salt (< 5cP), at high density (>1.3 g/ml; RG). One representative example of a suitable medium is ReadyGrade® (in general denoted RG and available from Amersham Biosciences, Uppsala, Sweden). Alternatively, silica particles can be bought from commercial sources, such as Nyacol, and silanised according to well known techniques by the user. The unique properties of the medium make it possible to retain the native characterstics of substances after separation. Further details regarding the medium will be given below. Accordingly, the present method is advantageous as compared to the prior art since it can reduce costs to a substantial degree.

The solution in b) is preferably an aqueous solution, such as a buffer solution.
The density in a) is higher than that of the desired substance(s) to be separated. In the present context, the skilled person will understand that the terms "low density" and "high density" are used in relation to each other and are not intended to be compared with any other densities. Illustrative densities will be given below.

In the method of the invention, the steps (i)-(iv) are repeated once or more depending on which substance is sought after or which contaminants are to be removed. The g-force in the centrifugation step(s) and the density of the colloidal media are varied according to the substance(s) to be separated.
The desired substance(s) to be separated is/are cells, micro-organisms, virus, and fractions thereof, nucleic acids and any proteins. Before centrifugation, the sample is processed into a mixture with the medium in order to provide an appropriate density thereof. The sample may be of practically any origin, such as a biological sample, e.g. cells, blood etc, faeces, e.g. to test for food poisoning, such as salmonella bacteria, foodstuffs, samples useful to analyse the environment, e.g. suspected pollutions or genetically manipulated organisms, etc. Accordingly, a great advantage of the present method is that it is applicable on much more complex samples than the prior art. The invention can be applied within a wide range of fields, such as for clinical analyses, forensic medicine, routine testing and many more. A few specific fields where the present method is especially advantageous will be described in the last paragraph of the present specification.

According to an alternative embodiment of the method, the desired substance(s) is/are derivatized before centrifugation to improve separation of two substances of similar density. An example of this is to treat the desired substance(s) with an affinity reagent linked to a high or low-density particle, such as mAb coated beads of suitable density.

In one specific embodiment, the method described above is specifically adapted to separate different cell types, such as X- and Y-sperm cells, from each other in a sample on the basis of their buoyant density, shape, permeability, movement, and/or viscosity in a density gradient medium by centrifugation. The method comprises the subsequent steps according to claim 1, with the sample being a semen sample and the desired substances being the separated cells.

Even though in principle any density medium capable of forming the above mentioned types of gradient for X and Y sperm separation could be used, the density medium of this embodiment is the kind of colloidal density medium discussed above.

The sample is mixed with high density medium and, optionally, said medium in step A) is centrifuged to form a density gradient before said sample is added to said centrifuge tube. The gradient is preferably very flat or essentially planar, i.e. it comprises a very small density difference in the centrifuge tube, at least at the centre of the gradient.

In this embodiment, the gradient is discontinuous. It is important that the gradient provides the necessary separation which means that it should be possible to recover fractions where X and Y sperms, respectively, are enriched to at least 70% or more, preferably 100%.

Optionally, the semen sample is purified before separation. This purification may be in any desired way, such as by discontinuous centrifugation.

When the sample is bovine, the density referred to above is close to that of the X and Y sperm cells, i.e. about 1.120g/mL. The corresponding values for human cells are substantially the same. These densities are applicable under the experimental conditions mentioned below in the experimental part. The present invention does not relate to centrifugations during which human X- and Y-sperm cells, respectively, have densities centred around 1.185 g/mL.

In the above method, the separation may be improved by manipulating the density of X and Y sperm cells, for instance by altering pH, conductivity of the sample and/or medium. The manipulation may comprise swelling of X- and Y-sperm cells.

All the above mentioned centrifugations for separation of X and Y cells from each other in a density gradient as defined above will be performed depending on choice of gradient solution, gradient shape, centrifuge rotor, centrifugation time, g-force at known viscosity, osmolarity and concentration levels.

The invention also relates to a method for separation as above, wherein said sample is mixed with said medium to achieve a density of said mixed sample-medium which lies close to the density of X and Y sperm cells. In this case, the separation is mainly achieved by isopycnic centrifugation. The separation pattern obtained in this variant of the invention will thus preferentially be based on the different buoyant densities of the sperm cells.

The colloidal density gradient medium comprises a suspension of particles having an average particle size of 2-40nm, preferably a hydrated particle of 10-30 nm.
Preferably the particles are derivatized silica particles, and more preferably silanised silica particles.

### Detailed description of the drawing

Fig. 1 represents a schematic view of the working principle of the method according to the present invention. In the example shown in the drawing, four centrifugation steps have been performed. The number of centrifugation steps and g-force is varied according to the nature of the substances to be separated.

### EXPERIMENTAL PART

The invention will now be described in association with the examples below, which are not to be interpreted as limiting the scope of the present invention as defined by the appended claims.

### General exemplifying non-limiting description:

The sample, mixed with RG (an example of one colloidal medium according to the invention) to a density of 1.057 g/ml, is placed at the bottom of a centrifuge tube, is optionally overlaid with RG having a density of 1.030 g/ml and on top of this a solution of 0.150 M NaCl is applied. Thereafter the tube is centrifuged 1 to 5 minutes at 10,000 x gₐᵥ (1, Figure 1). The resulting lower phase, resuspended and mixed with RG to a density of 1.200 g/ml, is placed at the bottom of a new centrifuge tube, is optionally overlaid with RG having a density of 1.090 g/ml and on top of this a solution of 0.150 M NaCl is applied. Then the tube is centrifuged for 1 to 5 minutes at 1,000 x gₐᵥ. Any cells and protozoans from the sample are recovered in the uppermost phase layer (2, Figure 1). The resulting lower phase is resuspended and transferred to a new centrifuge tube, is optionally overlaid with RG having a density of 1.130 g/ml and on top of this a solution of 0.150 M NaCl is applied. Then the tube is centrifuged for 1 to 5 minutes at 10,000 x gₐᵥ. Any bacteria from the sample are now recovered in the uppermost phase layer (3, Figure 1). The resulting lower phase is resuspended and transferred to a new centrifuge tube, is optionally overlaid with RG having a density of 1.130 g/ml and on top of this a solution of 0.150 M NaCl is applied. Then the tube is centrifuged for 1 to 5 minutes at 50,000 x gₐᵥ. Any viruses from the sample are now recovered in the uppermost phase layer (4, Figure 1).

By placing the sample, mixed with RG, at the bottom of the centrifuge tube, in contrast to the known techniques, all contamination problems associated with application of a heterogeneous sample solution on top of another solution are avoided.

According to the new method of the invention, the light density material in the sample matrix is floating in step 1 (Figure 1). This means that the cells/micro-organisms no longer have to sediment through a possibly formed plug but will in this step remain in the lower phase because of their density (ρ> 1.057). Thus, the average hydrostatic pressure resulting from centrifugation will be higher than in prior art techniques. This is due to that the distance to the rotational centre is longer when the sample is in the bottom of the tube as compared to when the sample is loaded on the top. This fact contributes to an increased yield when employing the present method compared with prior art.

In steps 2, 3, and 4 (Figure 1) desirable cells, bacteria and viruses are floating from the underlying sample phase and are conveniently recovered from the uppermost phase layer. Hereby all manipulations with the sample phase is avoided and thus recontamination with e.g. inhibitory activities from the sample solution is eliminated.

Since the sample containing cells / micro-organisms is located in the lower part of the centrifugation tube in every centrifugation step, the sedimentation towards the wall or bottom of the tube of the cells/micro-organisms is avoided. This reduces the cell damage frequency and increases the yield as compared to prior art techniques.

With this new method it has also been possible to purify viruses by discontinuous density centrifugation. Prior art techniques have used too large colloidal particles in the density media. The particles have sedimented as quickly as the viral particles at the higher g-forces needed for sedimentation/flotation of virus and thereby dissolved the phase boundaries. Also, the density of the known density media has been too low to allow mixing of the sample and the medium to a density of 1.200 g/ml, which is a requirement for this separation.

The following examples are provided to illustrate but not to limit the invention:

### Example 1 - Detection of E. coli in minced meat

To 10 g of locally purchased minced meat 90 ml of 0.15 M NaCl was added and then the sample was homogenised. Varying amounts of E. coli (10⁴, 10³, 10², 10¹) in 50 µl of 0.15 M NaCl were mixed with 0.5 g homogenised sample (=spiked samples) and 0.45 ml of 0.15 M NaCl (=control), respectively. The spiked samples were left to stand for 30 minutes before use so that any binding of the bacteria to the food matrix could occur. Spiked samples and corresponding controls were slurred and mixed with 85µl of RG to a final density of 1.057 g/ml. The mixture was transferred to the bottom of a centrifuge tube (1.5 ml), overlaid with 0.5 ml of a RG solution with a density of 1.030 g/ml and on top of this with 0.2 ml of a 0.15 M NaCl solution. Then, centrifugation was performed in a table centrifuge at 10,000 x gₐᵥ for 1 minute. Then, the lower phase was aspirated into a syringe whereby the cannula first penetrated the unpipettable plug formed by the minced meat matrix and was transferred to a new tube. No visible pellet was observed but to obtain any microscopic amounts of pelleted material (with bound bacteria) the remaining 100 µl was resuspended before being drawn into the syringe. The lower phase thus obtained was mixed with 585 µl RG to obtain a density of 1.200 g/ml, then overlaid with 0.2 ml of a RG solution with a density of 1.090 g/ml and on top of this 0.1 ml of a 0.15 M NaCl solution was added. This was centrifuged at 1,000 x gₐᵥ for 1 minute. The upper phases were aspirated with a Pasteur pipette and discarded and then the lower phase was resuspended, transferred to a new tube, overlaid with 0.2 ml of a RG solution with a density of 1.130 g/ml and 0.1 ml of a 0.15 M NaCl solution. Then, centrifugation was performed at 10,000 x gₐᵥ for 1 minute. The upper phases were fractionated with an automatic pipette in three portions of 75 µl and then analysed.

For comparison with prior art techniques, spiked samples and controls were loaded on top of 0.6 ml of a Percoll® solution in 0.15 M NaCl, pH 7.40 with a density of 1.057 and on top of 0.6 ml of BacXtractor (ρ= 1.057, 0.15 M NaCl, pH 7.40), respectively. Then, the samples were centrifuged at 10,000 x gₐᵥ. The lower 100 µl of the lower phase from each sample and control was drawn into a syringe and analysed. For determination of the amount of E. coli by culturing, 2 x 10 µl of varying dilutions from the different samples were spread on plates. Manual counting of the colonies and calculation of the amount were performed after incubation over night. For detection of E. coli by a DNA technique 10 µl from each sample was used in a PCR test.

According to the method of the invention the yield with the spiked samples was 80-100% as compared to 50-80% with the two older techniques. The yield in the controls was somewhat lower in every case as compared to the samples. This phenomenon has been reported earlier and is very likely due to the protective effect, which the endogenous flora in the minced meat has on the bacteria added in the samples.

By the PCR technique as little as 1.3 cfu E. coli per reaction tube (10 cfu/0.5 g homogenised sample; 200 cfu/g minced meat) could be detected using the method of the invention while down to 10 cfu E. coli per analysis (100 cfu/0.5 g homogenised sample; 2,000 cfu/g minced meat) could be detected using prior art methods for density gradient centrifugation. Direct analysis did not give any positive reaction. In the controls 1.3 cfu E. coli per reaction tube could be detected using either technique.

### Example 2 - Detection of E. coli in faeces

To 10 g human faeces 90 ml of 0.15 M NaCl was added and the sample was homogenised. Varying amounts of E. coli (10⁴, 10³, 10², 10¹) in 50 µl of 0.15 M NaCl were mixed with 0.5 g homogenised sample (=spiked samples) and with 0.45 ml of 0.15 M NaCl (=control), respectively. The spiked samples were left to stand for 30 minutes before use so that any binding of the bacteria to the food matrix could occur. Spiked samples and corresponding controls were slurred and mixed with 85µl of RG to a final density of 1.057 g/ml. The mixture was transferred to the bottom of a centrifuge tube (1.5 ml), overlaid with 0.5 ml of a RG solution with a density of 1.030 g/ml and on top with 0.2 ml of a 0.15 M NaCl solution. Then, centrifugation was performed in a table centrifuge at 10,000 x gₐᵥ for 1 minute. Thereafter the lower phase was aspirated into a syringe, whereby the cannula first penetrated the unpipettable plug formed by the sample matrix, and was transferred to a new tube. A small pellet was observed and to obtain the pelleted material (with bound bacteria) the remaining 100 µl was resuspended before being drawn into the syringe. The lower phase thus obtained was mixed with 585 µl RG to obtain a density of 1.200 g/ml, then overlaid with 0.2 ml of a RG solution with a density of 1.090 g/ml and on of this top 0.1 ml of a 0.15 M NaCl solution was added. Then centrifugation was performed at 1,000 x gₐᵥ for 1 minute. The upper phases were aspirated with a Pasteur pipette and discarded and then the lower phase was resuspended, transferred to a new tube, overlaid with 0.2 ml of a RG solution with a density of 1.130 g/ml and with 0.1 ml of a 0.15 M NaCl solution. Then, centrifugation was performed at 10,000 x gₐᵥ for 1 minute. The upper phases were fractionated with an automatic pipette in three portions of 75 µl and then analysed.

For comparison with prior art technique spiked samples and controls were loaded on top of 0.6 ml of a Percoll® solution in 0.15 M NaCl, pH 7.40 with a density of 1.057 and on top of 0.6 ml of BacXtractor (p= 1.057, 0.15 M NaCl, pH 7.40), respectively. Then, the samples were centrifuged at 10.000 x gₐᵥ. The lower 100 µl of the lower phase from each sample and control was drawn into a syringe and analysed.
For determination of the E. coli amount by culturing, 2 x 10 µl of varying dilutions from the different samples were spread on plates. Manual counting of the colonies and calculation of the amount were performed after incubation over night. For detection of E. coli with a DNA technique, 10 µl from each sample was used in a PCR test.

According to the method of the invention the yield with the spiked samples was 80-100% as compared to 50-80% with the two older techniques. The yield in the controls was somewhat lower in every case as compared to the samples. This phenomenon has been reported earlier and is very likely due to the protective effect, which the endogenous flora in the faeces sample has on the bacteria added in the samples.

By the PCR technique as little as 1.3 cfu E. coli per reaction tube (10 cfu/0.5 g homogenised sample; 200 cfu/g faeces) could be detected using the method of the invention while down to 10 cfu E. coli per analysis (100 cfu/0.5 g homogenised sample; 2,000 cfu/g faeces) could be detected using prior art methods for density gradient centrifugation. Direct analysis did not give any positive reaction. In the controls 1.3 cfu E. coli per reaction tube could be detected using either technique.

### Example 3 - Detection of E. coli in soil

To 10 g of composted soil 90 ml of 0.15 M NaCl was added and then the sample was homogenised. Varying amounts of E. coli (10⁴, 10³, 10², 10¹) in 50 µl of 0.15 M NaCl were mixed with 0.5 g homogenised sample (=spiked samples) and with 0.45 ml of 0.15 M NaCl (=control), respectively for analysis by the new technique. To obtain samples useful for comparison with the prior art techniques, the homogenised sample is first left to self sediment for 60 minutes and then the upper liquid was used to produce spiked samples as described above.
The spiked samples were left to stand for 30 minutes before use so that any binding of the bacteria to the sample matrix could occur. Spiked samples and corresponding controls were slurred and mixed with 85µl of RG to a final density of 1.057 g/ml. The mixture was transferred to the bottom of a centrifuge tube (1.5 ml), overlaid with 0.5 ml of a RG solution with a density of 1.030 g/ml and on top of this with 0.2 ml of a 0.15 M NaCl solution. Then, centrifugation was performed in a table centrifuge at 10,000 x gₐᵥ for 1 minute. The upper phases were aspirated with a Pasteur pipette and discarded. A large pellet was formed and to obtain all the pelleted material (with bound bacteria) the lower phase was resuspended before transferring it to a new tube. Thus, the lower phase obtained was mixed with 585 µl RG to obtain a density of 1.200 g/ml, then overlaid with 0.2 ml of a RG solution with a density of 1.090 g/ml and on top 0.1 ml of a 0.15 M NaCl solution was added. Then centrifugation was performed at 1,000 x gₐᵥ for 1 minute. The upper phases were aspirated with a Pasteur pipette and discarded and then the lower phase including the pellet was resuspended, transferred to a new tube, overlaid with 0.2 ml of a RG solution with a density of 1.130 g/ml and 0.1 ml of a 0.15 M NaCl solution. Then, centrifugation was performed at 10,000 x gₐᵥ for 1 minute. The upper phases were fractionated with an automatic pipette in three portions of 75 µl and then analysed.

For comparison with prior art techniques, specially prepared spiked samples and controls were loaded on top of 0.6 ml of a Percoll® solution in 0.15 M NaCl, pH 7.40 with a density of 1.057 and on top of 0.6 ml of BacXtractor (p= 1.057, 0.15 M NaCl, pH 7.40), respectively. Then, the samples were centrifuged at 10,000 x gₐᵥ. The upper phases were aspirated and discarded and then the lower phase was aspirated until 100 µl remained. The liquid above the formed pellet (60 µl) was aspirated and analysed.

For determination of the amount of E. coli by culturing, 2 x 10 µl of varying dilutions from the different samples were spread on plates. Manual counting of the colonies and calculation of the amount were performed after incubation over night. For detection of E. coli by a DNA technique 10 µl from each sample was used in a PCR test.

By the new technique the yield with the spiked samples was 80-100% as compared to 10-20% with the two older techniques. The yield in the controls was somewhat lower in every case as compared to the samples. This phenomenon has been reported earlier and is very likely due to the protective effect, which the endogenous flora in the soil sample has on the added bacteria in the samples.

By the PCR technique as little as 1.3 cfu E. coli per reaction tube (10 cfu/0.5 g homogenised sample; 200 cfu/g soil) could be detected using the method of the invention while down to 10 cfu E. coli per analysis (1,000 cfu/0.5 g homogenised sample; 20,000 cfu/g soil) could be detected using prior art methods for density gradient centrifugation. Direct analysis did not give any positive reaction. In the controls 1.3 cfu E. coli per reaction tube could be detected using either technique.

### Example 4 - Purification of sperms from bacteria and viruses in bovine semen

A fresh semen sample from a healthy bull was divided after dilution by 1:10 with 0.15 M NaCl, into 16 fractions of 0.45 ml each containing about 10⁷ sperms. Varying amounts of BVDV (bovine virus diarrhoea virus; 10⁶, 10⁵, 10⁴) and Campylobacter fetus (10⁶, 10⁵, 10⁴), respectively, in 50 µl of 0.15 M NaCl were added to 12 of the tubes and to four of the tubes 50 µl of 0.15 M NaCl were added as control.

Spiked samples (6 tubes) and controls (2 tubes) were mixed with 85 µl of RG to a final density of 1.057 g/ml. The mixture was transferred to the bottom of a centrifuge tube (3.0 ml), overlaid with 0.5 ml of a RG solution with a density of 1.030 g/ml and on top with 0,5 ml of a 0.15 M NaCl solution. Then, centrifugation was performed in a swing-out rotor at 10,000 x gₐᵥ for 1 minute. The upper phases were aspirated by a Pasteur pipette and discarded. The lower phase was resuspended, transferred to a new tube and mixed with 585 µl RG to obtain a density of 1.200 g/ml, overlaid with 0.5 ml of a RG solution with a density of 1.090 g/ml and on top with 0.5 ml of a 0.15 M NaCl solution. Then centrifugation was performed at 1,000 x gₐᵥ for 1 minute. The upper phases were aspirated by a Pasteur pipette and discarded and then the lower phase was resuspended, transferred to a new tube, overlaid with 0.5 ml RG solution with a density of 1.130 g/ml and with 0.5 ml of a 0.15 M NaCl solution. Then centrifugation was performed at 1,000 x gₐᵥ for 1 minute. Fractions were collected from the tubes from above with an automatic pipette in four portions of 300 µl and two fractions of 0.5 ml. The fractions were analysed regarding sperms, BVDV and Campylobacter fetus.

For comparison with prior art technique, samples (6 tubes) and controls (2 tubes) were stored respectively on top of a discontinuous gradient consisting of 0.5 ml of 80% and 0.5 ml of 40% SpermXtractor, respectively. Then, the samples were centrifuged at 400 x gₐᵥ for 10 minutes. The gradients were fractionated from above by a Pasteur pipette into 6 fractions (the upper phase, the first interface, the second phase, the second interface, the third phase, and the pellet). The fractions were analysed regarding sperms, BVDV and Campylobacter fetus.

For detection of the amount of Campylobacter fetus by culturing, 2 x 10 µl of varying dilutions from the different samples were spread on plates. Manual counting of the colonies and calculation of the level were performed after incubation over night. For detection of BVDV by immunological technique, 10 µl from each sample was used in an ELISA test.

By the new technique neither Campylobacter fetus nor BVDV in the three upper sperm containing fractions from the spiked samples were detected. In contrast, BVDV and Campylobacter fetus were detected in the lower sperm free fractions. The controls were negative for these agents.

Using prior art techniques, detectable amounts of both BVDV and Campylobacter fetus were present in all fractions of the spiked samples including fractions with enriched intact sperms even though most of the organisms remained in the uppermost fraction. The controls were negative for these agents.

### Further applications of the invention

The method of the invention may also be used for, for example testing of bacterial content in an enzyme fraction from a fermentation process. Another example is purification of dissolved substances (toxins, antibiotics, hormones) from food stuff. Following aspiration of the suitable layer analysis is performed by GC-MS, ELISA, Dip-Stick etc.

A further example is purification of dissolved substances (toxins, antibiotics, hormones) from food stuff by mixing the sample before centrifugation with mAb-coated beads of suitable density and analysing after loosening by GC-MS, ELISA, Dip-Stick etc. The invention may also be used in criminal investigation/forensic medicine, for example purification of DNA from bloodstains or purification of cells/DNA from vacuumed samples. Another example is purification of sperms/DNA from stains/clinical samples. The invention is also useful for purification of nucleic acids and proteins for analytical and/or preparative purposes.

The extraordinary purity of a separated substance obtained by the present invention makes it especially advantageous for all applications where high purity is a requirement, such as in most purification of DNA and RNA. Even ribosomes and other cell components can be obtained as true solutions by an appropriate design of the present method. It is also advantageous in the subsequent handling of such samples since they are normally subject to PCR, in which case a first step is lysis.

The present invention can also be used to separate living cells from dead cells, in which case the cell type can be the same, since properties change when the cell cease to live. More specifically, the present method can be used to differentiate between active and not active cells. In some cases, cells are viable but still not culturable, and such can now be separated from dead cells. In this context, it is essential that the density of a living cell is not an absolute value, but will vary depending on its environment, such as osmolality (salt level). Thus, by an appropriate manipulation of the environment, the density of the living cells can be changed, but the dead cell will keep their density, and accordingly the present method will be readily applicable.

An unexpected use of the present method is in the analysis of used swab tests, commonly used in slaughter houses. It has been shown that the present method is applicable to such a swab, without a prior enrichment step. This makes it possible to detect pathogens in such samples in a rapid and cost-effective way.

## Claims

1. A method of discontinuous density gradient centrifugation for separating at least two substances from each other in a sample, which method comprises the subsequent steps of
(i) mixing the sample with a high density medium to obtain a specific density of the mixture;
(ii) placing
a) the mixture of a high density centrifugation medium and the sample comprising the substances to be separated at the bottom of a centrifuge tube, in which mixture the density is defined and is higher than that of the desired substance(s) to be separated,
b) a solution of low density on top of the mixture in a);
(iii) centrifuging said tube under a defined g-force so to allow the desired substance(s) or solute(s) in said sample to float to the low density solution whereas other substance(s) in said sample remain in the bottom denser media;
(iv) collecting separately the desired substance(s),
wherein the centrifugation medium is a colloidal density gradient medium.

2. A method according to claim 1, wherein a colloidal density gradient centrifugation medium of a lower density than the density of the mixture in a) is placed between the mixture in a) and the low density solution in b).

3. A method according to claim 1 or 2, wherein the solution in b) is an aqueous solution.

4. A method according to any of the above claims, wherein the colloidal density gradient medium comprises a suspension of particles having an average particle size of 2-40 nm, preferably a hydrated diameter of 10-30 nm.

5. A method according to any of the above claims, wherein the lower phase is collected after step (iv) and resuspended to provide a second mixture of a defined density, with which second mixture steps (i)-(iv) are repeated, wherein both the density of the second mixture and/or the g-force are different during the repetition of steps (i)-(iv) as compared to the previous round of steps (i)-(iv).

6. A method according to claim 5, wherein at least the steps (ii)-(iv) are repeated any number of times depending on the nature of the desired substance.

7. A method according to any of claims 5 or 6, wherein each combination of a g-force during the centrifugation step and a defined density of the colloidal medium is selected for each round of steps (i)-(iv) to enable separation of one or more desired substance(s).

8. A method according to any of the above claims, wherein the desired substance(s) to be separated is/are cells, micro-organisms, virus, and fractions thereof, nucleic acids and proteins.

## Patentansprüche

1. Verfahren zur diskontinuierlichen Dichtegradientenzentrifugation zum Trennen mindestens zweier Substanzen voneinander in einer Probe, wobei das Verfahren die nachfolgenden Schritte umfaßt:
(i) Mischen der Probe mit einem hochdichten Medium, um eine spezifische Dichte der Mischung zu erhalten;
(ii) Anordnen
(a) der Mischung eines Hochdichtezentrifugationsmediums und der Probe, die die zu trennenden Substanzen umfaßt, auf dem Boden eines Zentrifugenröhrchens, wobei in der Mischung die Dichte definiert ist und höher ist als jene der zu trennenden erwünschten Substanz(en),
(b) einer Lösung niedriger Dichte oben auf der Mischung in (a);
(iii) Zentrifugieren des Röhrchens derart unter einer definierten g-Kraft, daß der/den erwünschte(n) Substanz(en) oder dem/den gelöste(n) Stoff(en) in der Probe ermöglicht wird, auf der Niedrigdichtelösung zu schwimmen, während (eine) andere Substanz(en) in der Probe in dem unteren dichteren Medium bleibt/bleiben;
(iv) getrenntes Sammeln der erwünschten Substanz(en);
wobei das Zentrifugationsmedium ein kolloidales Dichtegradientenmedium ist.

2. Verfahren nach Anspruch 1, wobei ein kolloidales Dichtegradientenzentrifugationsmedium einer niedrigeren Dichte als die Dichte der Mischung in (a) zwischen der Mischung in (a) und der Niedrigdichtelösung in (b) angeordnet wird.

3. Verfahren nach Anspruch 1 oder 2, wobei die Lösung in (b) eine wäßrige Lösung ist.

4. Verfahren nach einem der oben stehenden Ansprüche, wobei das kolloidale Dichtegradientenmedium eine Suspension von Teilchen mit einer durchschnittlichen Teilchengröße von 2 bis 40 nm, bevorzugt einem hydrierten Durchmesser von 10 bis 30 nm, umfaßt.

5. Verfahren nach einem der oben stehenden Ansprüche, wobei die untere Phase nach dem Schritt (iv) gesammelt und resuspendiert wird, um eine zweite Mischung einer definierten Dichte bereitzustellen, mit der die zweiten Mischungsschritte (i)-(iv) wiederholt werden, wobei sowohl die Dichte der zweiten Mischung als auch/oder die g-Kraft während der Wiederholung der Schritte (i)-(iv) im Vergleich zur vorherigen Runde der Schritte (i)-(iv) verschieden ist.

6. Verfahren nach Anspruch 5, wobei mindestens die Schritte (ii)-(iv) eine beliebige Anzahl von Malen abhängig von der Natur der erwünschten Substanz wiederholt werden.

7. Verfahren nach einem der Ansprüche 5 oder 6, wobei jede Kombination einer g-Kraft während des Zentrifugationsschrittes und einer definierten Dichte des kolloidalen Mediums für jede Runde von Schritten (i)-(iv) ausgewählt wird, um die Trennung einer oder mehrerer erwünschter Substanz(en) zu ermöglichen.

8. Verfahren nach einem der oben stehenden Ansprüche, wobei die zu trennende(n) erwünschte(n) Substanz(en) Zellen, Mikroorganismen, Virus, und Bruchteile davon, Nukleinsäuren und Proteine ist (sind).

## Revendications

1. Procédé de centrifugation sur gradient de densité discontinu destiné à séparer au moins deux substances l'une de l'autre dans un échantillon, le procédé comprenant les étapes consécutives de
(i) mélange de l'échantillon avec un milieu de densité élevée afin d'obtenir une densité spécifique pour le mélange ;
(ii) mise en place
a) du mélange d'un milieu de centrifugation de densité élevée et de l'échantillon comprenant les substances à séparer au fond d'un tube de centrifugation, la densité de ce mélange étant définie et étant supérieure à celle de la ou des substances que l'on désire séparer,
b) d'une solution de faible densité au-dessus du mélange mis en place en a) ;
(iii) centrifugation dudit tube sous une accélération définie afin de permettre à la ou aux substances ou solutés désirés dans ledit échantillon de flotter sur la solution de faible densité alors qu'une ou plusieurs autres substances dans ledit échantillon restent dans les milieux inférieurs plus denses ;
(iv) collecte de la ou des substances désirées de manière distincte,
dans lequel le milieu de centrifugation est un milieu colloïdal à gradient de densité.

2. Procédé selon la revendication 1, dans lequel un milieu de centrifugation colloïdal à gradient de densité d'une densité plus faible que la densité du mélange mis en place en a) est placé entre le mélange mis en place en a) et la solution de faible densité mise en place en b).

3. Procédé selon la revendication 1 ou 2, dans lequel la solution obtenue en b) est une solution aqueuse.

4. Procédé selon l'une quelconque des revendications précédentes, dans lequel le milieu colloïdal à gradient de densité comprend une suspension de particules présentant une taille moyenne de particule de 2 à 40 nm, de préférence un diamètre hydraté de 10 à 30 nm.

5. Procédé selon l'une quelconque des revendications précédentes, dans lequel la phase inférieure est collectée après l'étape (iv) et remise en suspension afin de former un deuxième mélange d'une densité définie, les étapes (i) à (iv) étant répétées avec ce deuxième mélange, dans lequel à la fois la densité du deuxième mélange et/ou l'accélération sont différentes au cours de la répétition des étapes (i) à (iv) par comparaison au précédent cycle d'exécution des étapes (i) à (iv).

6. Procédé selon la revendication 5, dans lequel au moins les étapes (ii) à (iv) sont répétées un nombre quelconque de fois en fonction de la nature de la substance désirée.

7. Procédé selon l'une quelconque des revendications 5 ou 6, dans lequel chaque association d'une accélération au cours de l'étape de centrifugation et d'une densité définie du milieu colloïdal est sélectionnée pour chaque cycle d'exécution des étapes (i) à (iv) afin de permettre la séparation d'une ou plusieurs substances désirées.

8. Procédé selon l'une quelconque des revendications précédentes, dans lequel la ou les substances que l'on désire séparer sont des cellules, des micro-organismes, des virus, et des fractions de ceux-ci, des acides nucléiques et des protéines.
